# FASCICULE DE BREVET EUROPEEN

(11) **EP 1 992 687 B1**
(45) Date de publication et mention de la délivrance du brevet: **26.06.2019**
(21) Numéro de dépôt: 08163979.1
(22) Date de dépôt: 07.03.2003
(51) Int. Cl.: C12N 5/0735, C12N 5/10

(54) **Lignées de cellules aviaires utiles pour la production de substances d'intérêt**
Aviäre Zelllinien für die Herstellung von nützlichen Substanzen
Avian cell lines for producing useful substances

(30) Priorité: 08.03.2002 FR 0202945
(43) Date de publication de la demande: 19.11.2008
(62) Demande divisionnaire de: 03725273.1
(73) Titulaire: Valneva, 44800 Saint Herblain (FR)
(72) Inventeur: Pain, Bertrand, 63830 Nohanent (FR); Guehenneux, Fabienne, 44360 le temple de Bretagne (FR)
(74) Mandataire: Flesselles, Bruno F.G.

(56) Documents cités:
- WO-A-00/47717
- WO-A-96/12793
- WO-A-99/06534
- US-A- 5 656 479
- PAIN B ET AL: "LONG-TERM IN VITRO CULTURE AND CHARACTERIZATION OF AVIAN EMBRYONIC STEM CELLS WITH MULTIPLE MORPHOGENETIC POTENTIALITIES" DEVELOPMENT, COMPANY OF BIOLOGISTS, CAMBRIDGE,, GB, vol. 122, 1996, pages 2339-2348, XP002164792 ISSN: 0950-1991
- PAIN B ET AL: "Chicken embryonic stem cells and transgenic strategies." CELLS TISSUES ORGANS, vol. 165, no. 3-4, 1999, pages 212-219, XP000882175 ISSN: 1422-6405
- KARAGENC L ET AL: "Soluble factors and the emergence of chick primordial germ cells in vitro." POULTRY SCIENCE, vol. 79, no. 1, janvier 2000 (2000-01), pages 80-85, XP008010610 ISSN: 0032-5791
- ACLOQUE H ET AL: "IDENTIFICATION OF A NEW GENE FAMILY SPECIFICALLY EXPRESSED IN CHICKEN EMBRYONIC STEM CELLS AND EARLY EMBRYO" MECHANISMS OF DEVELOPMENT, ELSEVIER SCIENCE IRELAND LTD, IE, vol. 103, no. 1/02, 1 janvier 2001 (2001-01-01), pages 79-91, XP001015571 ISSN: 0925-4773
- BERGER C N ET AL: "Self renewal of embryonic stem cells in the absence of feeder cells and exogenous leukaemia inhibitory factor." GROWTH FACTORS (CHUR, SWITZERLAND) SWITZERLAND 1997, vol. 14, no. 2-3, 1997, pages 145-159, XP008010580 ISSN: 0897-7194

## Description

La présente description décrit un procédé d'obtention de lignées cellulaires aviaires, notamment des cellules souches aviaires, comprenant un sevrage progressif ou total des facteurs de croissance, du sérum et/ou du tapis nourricier. Ces lignées établies spontanément sont des cellules adhérentes ou non-adhérentes capables de proliférer indéfiniment dans un milieu de culture de base. L'invention porte sur les cellules dérivant de telles lignées qui sont particulièrement utiles pour la production de vaccins et de substances d'intérêt.

Les cellules souches sont des cellules identifiées par leur mise en culture *in vitro* à partir d'un embryon, d'une partie d'un embryon ou même d'un tissu adulte. Par cellule souche, on entend toute cellule pluripotente d'origine embryonnaire ou adulte qui présente une capacité d'autorenouvellement et capable de donner des cellules différenciées spécialisées. En d'autres termes, toute cellule non cancéreuse capable de se diviser indéfiniment en culture et de donner une cellule fille présentant la même capacité de prolifération et de différenciation que la cellule mère dont elle est issue. Ces cellules isolées présentent des caractéristiques morphologiques et immuno-cytochimiques particulières. On peut également distinguer la notion de :
- cellules souches embryonnaires (cellules CES), cellules souches qui ont la particularité d'être obtenues à partir de la mise en culture de parties ou de l'ensemble d'un embryon très précoce (stade blastula). Ces cellules CES présentent *in vitro* toutes les caractéristiques d'une cellule souche, et *in vivo* la capacité unique de contribuer à la morphogenèse d'un embryon et de participer à la colonisation germinale quand elles sont réimplantées de quelque manière que cela soit dans un embryon receveur ;
- cellules souches somatiques (CSS), cellules qui ont toutes les caractéristiques des cellules souches lorsqu'elles sont cultivées *in vitro,* mais qui contrairement aux cellules CES n'ont pas la potentialité de coloniser *in vivo* les gonades après injection dans un embryon. Elles contribuent uniquement à la morphogénèse des tissus somatiques dans l'embryon.

A l'inverse des cellules primaires déjà différenciées, les cellules souches ne présentent pas un état de différenciation morphologique caractéristique facilement identifiable (fibroblaste, adipocyte, macrophage, etc.), mais sont plutôt caractérisées par un état de prolifération et de non différenciation. Cet état se traduit par différents comportements comme une prolifération rapide *in vitro,* une morphologie caractéristique, la présence de différents marqueurs, des besoins en facteurs de croissance variables et une aptitude à répondre à des stimuli d'induction de différenciation particuliers. Elles ne sont pas sensibles à la sénescence réplicative, période critique pour un grand nombre de cellules primaires différenciées, dont les fibroblastes par exemple.

Pour le maintien *in vitro* des cellules souches aviaires pendant des périodes de temps importantes, il faut respecter des conditions de culture et d'entretiens spécifiques telles que décrites dans Pain et al., 1996, US 6,114,168 et EP 787 180.

La mise en culture *in vitro* d'une cellule primaire dans des conditions satisfaisantes de milieu et de facteurs de croissance lui permet de proliférer seulement pendant un certain nombre de passages. Cette cellule peut être obtenue directement d'un tissu dissocié ou d'une partie de ce tissu. Ce nombre de passages est néanmoins très dépendant de l'espèce animale considérée, de l'origine du tissu, de l'âge de l'organisme donneur, etc. Dans la plupart des cas, la prolifération cellulaire observée *in vitro* ralentit progressivement puis les cellules cessent de proliférer.

Cet arrêt correspond souvent à une sénescence réplicative, connue sous le terme de limite de Hayflick. Cet arrêt serait le résultat de l'action d'une véritable horloge moléculaire dont un des acteurs clefs serait la longueur des télomères. Les télomères sont des séquences répétées situées à l'extrémité des chromosomes. Le raccourcissement de ces structures nucléotidiques répétitives est la conséquence de la réplication du DNA sur un mode semi conservatif. En l'absence de l'enzyme télomérase, en charge d'ajouter les séquences répétées à l'extrémité des chromosomes, un point de non-retour est atteint quant à la taille des télomères, point au-delà duquel est déclenché un mécanisme moléculaire encore inconnu d'activation de gènes impliqués dans le contrôle du cycle cellulaire. Les cellules seraient alors bloquées en phase G1 dans leurs divisions et cesseraient de proliférer. De nombreux acteurs semblent intervenir dans ce contrôle négatif du cycle cellulaire comme les différentes cyclines, les kinases spécifiques, les protéines RB, P53, les facteurs de transcription spécifiques comme E2F et bien d'autres (mdm2, BTG, p21, ...). A l'inverse, l'enzyme télomérase peut donc être vue comme un acteur central de l'immortalité cellulaire, car elle maintient la longueur des télomères et donc rend insensible la cellule à cette perte engendrée par les divisions successives.

Dans un organisme en développement et au cours de la vie de cet organisme, seuls quelques types cellulaires, dont certains lymphocytes présentent une expression permanente de la télomérase. Cette activité semble également être une des caractéristiques des cellules souches, à la fois au niveau somatique (CSS) et au niveau germinal. Cette propriété d'expression, de maintien d'expression ou de 'réveil' d'expression de l'activité télomérase est également souvent associée au caractère immortel d'une cellule maintenue *in vitro.* A ce jour, de nombreuses cellules cancéreuses sont également détectées positives pour l'activité télomérase. Cette activité serait en partie responsable de la capacité de prolifération incontrôlée des cellules tumorales *in vivo.*

Cette activité télomérase est, dans tous les cas, un excellent marqueur du caractère cellule souche ainsi que du lignage germinal et de la capacité d'une cellule à devenir immortelle. Deux critères sont donc utilisés : l'activité de la télomérase et la taille des télomères.

Dans une perspective d'établissement et de façon très schématique, d'après la littérature et les résultats déjà disponibles dans de nombreux laboratoires, l'établissement de lignées cellulaires peut être réalisé selon deux voies : un établissement spontané résultant de dommages génétiques intrinsèques non induits ou un établissement provoqué, induit par l'utilisation de virus, rétrovirus ou par d'autres moyens tels que des agents chimiques, des irradiations, des U.V. (Ultra-Violet), .... Au niveau des mammifères, par exemple, l'établissement des cellules de rongeurs (souris, rat, ...) est reconnu comme assez facile de façon spontanée, par contre, la situation est toute autre pour les cellules humaines, quelle que soit leur origine tissulaire (Smith and Pereira-Smith, 1996).

Ainsi, lorsque l'on souhaite dériver des lignées cellulaires des cellules souches aviaires mentionnées ci-dessus en vue de produire en masse des substances d'intérêt *in vitro,* le problème est de pouvoir maintenir des cellules en culture dans un milieu économique tout en évitant les écueils tels que la différenciation et la sénescence cellulaire.

Au niveau aviaire, il est en général admis que l'établissement de cellules primaires est un événement rare, voire quasi inexistant. La seule exception notable publiée semble être la lignée DF-1 qui résulte de l'immortalisation décrite comme spontanée de fibroblastes de poulet (Foster et al., 1991 ; brevet US 5,672,485, N° ATCC CRL 12203). Au niveau de cette lignée, des articles récents mentionnent les premiers éléments de dérégulation observée (Kim et al., 2001a ; Kim et al., 2001b).

Dans le processus d'immortalisation, une première étape conduit la cellule en prolifération vers la limite de Hayflick, qui selon les types cellulaires se situe entre 10 et 50 passages. Un premier événement mutationnel spontané a alors lieu qui permet à la cellule de franchir ce premier blocage, événement qui affecte souvent les gènes p53, pRb, .... Les cellules continuent donc de proliférer jusqu'au moment où un deuxième blocage intervient qui est en général levé par de nouvelles mutations dans d'autres gènes et par l'activation de la télomérase, souvent observée.

Au niveau aviaire, il est en général admis que l'établissement de cellules primaires immortelles est un événement quasi inexistant. C'est pourquoi un nombre important de lignées ont été obtenues par la mise en culture de cellules tumorales, souvent directement prélevées à partir d'une biopsie de la tumeur. Cette obtention *in vitro* est en fait le résultat de l'altération *in vivo* de certains gènes, responsables de l'apparition de la tumeur. Un exemple est fourni par la lignée fibroblastique SB-CEV-1, isolée à partir de la mise en culture d'une tumeur viscérale d'un animal. (ATCC N° CRL 10497, brevet US N° 5,846,527). Cette approche est grandement facilitée par l'existence d'un très grand nombre de virus et rétrovirus aviaires, identifiés, isolés et souvent caractérisés au niveau moléculaire. Souvent oncogéniques par leur action directe ou indirecte (activation d'un gène endogène transformant lors de leur intégration, expression de la (ou les) protéine oncogénique endogène au virus), ces virus provoquent des tumeurs, des lésions, ainsi l'obtention de lignées cellulaires dans différents lignages différenciés est possible à partir de la mise en culture des organes infectés des animaux. L'utilisation *in vitro* de ces virus et rétrovirus, isolés *in vivo,* a été développée de façon complémentaire. On peut ainsi citer de façon non exhaustive :
- les lignées lymphoblastoïdes DT40 et DT95, obtenues en présence du virus de la leucose aviaire (ALV) et dans lesquelles le locus myc est activé (Baba et al., 1985, ATCC, N°CRL 2111, CRL N° 2112),
- la lignée lymphoblastoïde de dinde MDTC-RP19, établie avec le virus de la maladie de Marek (US N° 4,388,298, ATCC N° 8135),
- la lignée lymphoblastoïde ConA-C1, établie avec le virus REV (Reticuloendothelial virus, ATCC N° 12135, US N° 5,691,200),
- la lignée myélomonocytaire BM2 établie avec le virus MH2 (Liu et al., 1977, brevet US N°5,388,680),
- et toute une série de lignées hématopoiétiques obtenues avec différents virus,
   ∘ la lignée érythroblastique HD4 (6C2), obtenue avec le virus AEV ;
   ∘ la lignée monocytaire HD11 (Beug et al., 1979), obtenue avec le virus MC29;
   ∘ la lignée granulocytaire HD13 (Golay et al., 1988), obtenue avec le virus E26 ;
   ∘ la lignée hématopoiétique mixte HD57 (Metz and Graf, 1991) également obtenue avec le virus E26.

Cependant, le problème dans ces approches de transformation est l'obtention de cellules qui produisent des virus et portent le génome activé des virus et rétrovirus utilisés. Ces activations et cette présence virale sont des freins à leur utilisation industrielle comme support de réplication pour des virus d'intérêt ou pour la production de protéines spécifiques dans des conditions optimales de sécurité.

Une autre approche de sur-expression d'oncogènes, de gènes immortalisants (gène E1A de l'adénovirus, 'large T' du polyome SV40, etc.) ou de fragments de gènes a également permis d'obtenir des lignées à partir de cellules primaires déjà différenciées. Ces éléments peuvent être introduits dans les cellules par simple transfection de vecteur permettant l'expression de la partie immortalisante, mais également introduits via les virus ou rétrovirus modifiés génétiquement pour exprimer ces éléments immortalisants. L'origine des immortalisants peut être aviaire ou non, virale ou non. Le tropisme pour les cellules aviaires pouvant être d'ailleurs lié au virus originel ou également modifié. A titre d'exemple, la lignée TDF-2A de fibroblastes de canard est ainsi obtenue par introduction d'un premier gène immortalisant puis d'un gène anti-apoptotique (Guilhot et al., 1993 ; brevet US 6,255,108). D'autres méthodes ont été développées, comme la sur-expression de p53 (Foster et al. ; brevet US N° 5,830,723).

En outre, l'action de carcinogènes chimiques directement sur l'animal selon différents modes d'administration a permis entre autres l'obtention :
- des lignées QT6 et QT35, de fibroblastes de caille (Moscovici et al., 1977, ATCC N° CRL 1708),
- de la lignée d'hépatocyte de poulet LMH (Kawaguchi et al., 1989, ATTC N° CRL 2117),
- de la lignée CHCC-OU2 de fibroblastes de poulet (ATCC, N° CRL 12302, brevet US N° 5,989,805).

Par événement d'immortalisation, on entend différentes actions comme :
- l'action de stress oxydatifs, thermiques, chimiques capables d'induire des modifications de la physiologie des cellules et/ou des mutations,
- l'action des produits de gènes spécifiques dans la physiologie des cellules comme certains gènes immortalisants (oncogènes, proto-oncogènes, gènes du cycle cellulaire, gènes anti-apoptiques, etc.),
- la destruction ciblée par recombinaison ou inactivation fonctionnelle d'anti-oncogènes, de gènes apoptiques, de gènes suppresseurs de tumeurs, de gènes anti-prolifératifs menant à une dérégulation fonctionnelle du cycle cellulaire ou de la physiologie de la cellule,
- le contrôle de gènes de prolifération par leur blocage fonctionnel, etc.,
- l'action de rayonnements (UV, gamma, X, etc.),
- l'action de mutagènes chimiques (substances endommageant l'ADN, substances analogues de facteurs de croissance, etc.),
- l'action conjuguée de ces différentes actions prises séparément.

Dans le cadre de l'invention, on a trouvé que le sevrage des facteurs de croissance, du sérum et/ou du tapis nourricier conduit à l'isolement de populations de cellules souches, notamment de cellules souches somatiques, pouvant croître indéfiniment dans des milieux de culture de base.

En outre, hormis les cellules souches hématopoiétiques qui sont pour la plupart des cellules non adhérentes, la majorité des cellules, obtenues selon les techniques de l'art évoquées ci-dessus (fibroblastes, hépatocytes, etc.), présentent un phénotype adhérent. Or, l'utilisation industrielle des cellules, comme support de réplication virale, privilégie les cellules non adhérentes. Ce phénotype est intéressant tant pour des raisons de facilité de manipulation qui évite l'usage d'enzyme protéolytique de dissociation que pour les densités importantes atteintes par les cellules non-adhérentes cultivées *in vitro.*

La présente invention décrit l'obtention de lignées qui peuvent devenir non-adhérentes spontanément ou pour lesquelles la non adhérence a été obtenue par un sevrage du tapis nourricier. Du fait de leur croissance en suspension, ces lignées sont parfaitement adaptées à une utilisation industrielle pour la production de substances d'intérêt dans des bioréacteurs.

En plus de leurs propriétés à croître sur milieu de culture de base, on a découvert que ces lignées cellulaires permettent la réplication de certains virus à des rendements équivalents voire supérieurs aux rendements obtenus avec les procédés en cours ; ce qui rend ces cellules particulièrement utiles pour la production en masse de vaccins.

### Description

Ainsi, dans un premier aspect, la présente demande décrit un procédé d'obtention de lignées cellulaires aviaires, caractérisé en ce qu'il comprend les étapes suivantes :
a) mise en culture de cellules aviaires dans un milieu comportant l'ensemble des facteurs permettant leur croissance et un tapis nourricier inactivé (feeder) ;
b) passage en modifiant le milieu de culture de sorte à obtenir un sevrage progressif ou total desdits facteurs ;
c) établissement de lignées cellulaires adhérentes ou non-adhérentes capables de proliférer dans un milieu de base en l'absence de facteurs de croissance exogènes.

Dans le cadre de l'invention, on entend par « établissement d'une lignée », le maintien de cellules en culture *in vitro* sur une période considérable de temps. Avantageusement, les cellules issues des lignées obtenues à l'étape c) sont capables de proliférer pendant au moins 50 jours, 100 jours, 150 jours, 300 jours ou de préférence au moins 600 jours. Les 600 jours ne constituent pas une limite temporelle car les lignées cellulaires obtenues sont toujours vivantes après des périodes de temps bien plus longues. Dès lors, ces lignées sont considérées comme pouvant croître indéfiniment en milieu de culture de base dépourvu de facteurs de croissance exogènes, de sérum et/ou du tapis nourricier inactivé. Par « lignée », on entend toute population de cellules capables de proliférer indéfiniment en culture *in vitro* en gardant peu ou prou les mêmes caractéristiques morphologiques et phénotypiques.

Les cellules issues des lignées selon l'invention peuvent être des cellules souches aviaires, notamment des cellules souches somatiques aviaires.

Les cellules souches selon l'invention peuvent servir pour dériver des lignées de cellules différenciées. En effet, ces cellules souches présentent la propriété d'être pluripotentes, c'est-à-dire qu'elles ont les potentialités pour être induites dans de multiples voies de différenciation, caractérisables par différents marqueurs spécifiques.

Ces cellules peuvent également être des cellules précurseurs, qui correspondent aux cellules d'un tissu adulte ou embryonnaire partiellement différenciées par opposition à une cellule souche et qui est capable de se diviser et de donner des cellules plus différenciées. Par « cellule différenciée », on entend toute cellule d'un tissu adulte ou embryonnaire spécialisée, présentant des marqueurs spécifiques ou remplissant des fonctions physiologiques spécifiques. Il est possible, dans un aspect particulier de l'invention, notamment pour des isolats particuliers ou des clones issus d'un isolat particulier obtenu au cours de l'établissement, que ces cellules souches puissent contribuer à la lignée germinale. Dans ce cas, ces cellules souches établies en lignées seraient des cellules souches embryonnaires.

Bien entendu, le procédé mentionné ci-dessus permet d'obtenir des clones cellulaires issus des cellules provenant des lignées établies. Ces clones sont des cellules génétiquement identiques à la cellule de laquelle elles dérivent par division.

Dans un mode de réalisation particulier, l'invention est obtenue *via* un procédé tel que défini précédemment, et précisé ci-après dans lequel les lignées établies sont des cellules souches adhérentes qui prolifèrent aussi en l'absence du tapis nourricier inactivé.

A ce titre, dans le procédé décrit ci-dessus, l'étape b) consiste en un sevrage des composants du milieu (facteurs de croissance seulement ou facteurs de croissance puis sérum ou encore sérum puis facteurs de croissance).

Dans un autre mode de réalisation, l'invention est obtenue *via* un procédé tel que défini précédemment et précisé ci-après, dans lequel les lignées établies sont des cellules souches non adhérentes qui prolifèrent en suspension dans un milieu dépourvu de facteurs de croissance exogènes.

A ce titre, dans le procédé décrit ci-dessus, l'étape b) consiste en un sevrage progressif ou total du tapis nourricier en plus éventuellement du sevrage des autres composants du milieu (facteurs de croissance et sérum).

Dans un autre mode de réalisation, l'invention est obtenue *via* un procédé tel que défini précédemment et précisé ci-après, dans lequel les lignées établies sont des cellules souches non adhérentes qui prolifèrent en suspension dans un milieu également dépourvu de sérum (milieu asérique).

Dans un autre mode de réalisation, l'invention est obtenue *via* un procédé tel que défini précédemment et précisé ci-après, dans lequel les lignées établies sont des cellules souches non adhérentes qui prolifèrent en suspension dans un milieu dépourvu de facteurs de croissance exogènes et de sérum.

Dans une autre alternative, l'étape b) consiste en un sevrage progressif ou total des facteurs de croissance, optionnellement suivi par un sevrage progressif du sérum.

Dans une autre alternative, l'étape b) consiste en un sevrage progressif ou total des facteurs de croissance et du sérum, optionnellement suivi par un sevrage du tapis nourricier.

En outre, les lignées établies peuvent être des cellules qui prolifèrent dans un milieu appauvri en sérum, en particulier dans un milieu dépourvu de sérum. Par appauvri en sérum on entend une diminution progressive de la concentration en sérum étalée dans le temps. Cette méthode permet une sélection de clones qui s'adaptent à ces nouvelles conditions de plus en plus drastiques jusqu'à l'obtention de lignées stables qui sont capables de croître en milieu appauvri en sérum ou encore complètement dépourvu de sérum.

Le procédé décrit ci-dessus peut comprendre en outre une étape dans laquelle les cellules obtenues à l'étape c) sont soumises à une sélection dans des milieux de cultures utilisés pour la production à grande échelle de sorte à obtenir des clones adaptés à la production de vaccins destinés à la thérapie humaine ou animale.

Les cellules selon l'invention présentent au moins une des caractéristiques suivantes :
- un rapport nucléocytoplasmique important,
- une activité alcaline phosphatase endogène,
- une activité télomérase endogène,
- une réactivité avec des anticorps spécifiques sélectionnés parmi le groupe des anticorps SSEA-1 (TEC01), SSEA-3, et EMA-1.

De préférence, les cellules de l'invention présentent l'ensemble des caractéristiques mentionnées ci-dessus.

Dans un aspect supplémentaire, l'invention est obtenue *via* un procédé d'obtention de lignées aviaires mentionné ci-dessus et précisé ci-après, dans lequel les cellules issues des lignées obtenues à l'étape c) sont modifiées pour permettre une meilleure utilisation *in vitro* comme l'extension de la durée de vie ou des densités de croissance plus importantes ou encore des besoins en nutriments moins importants.

Avantageusement, les cellules issues des lignées établies sont modifiées pour produire une substance d'intérêt, notamment un polypeptide d'intérêt, un anticorps ou un virus atténué. Lesdites cellules peuvent être modifiées avec toute technique à la portée de l'homme du métier, notamment la recombinaison homologue, dirigée et/ou conditionnelle (système Cre-Lox ou FLP-FRT), par transformation par tout vecteur, plasmide, notamment à l'aide de rétrovirus.

Le milieu utilisé à l'étape a) peut comprendre au moins un facteur sélectionné parmi les cytokines, notamment LIF, IL-11, IL-6, IL-6R, CNTF, Oncostatine et d'autres facteurs tels que le SCF, l'IGF-1 et le bFGF.

En outre, le tapis nourricier inactivé utilisé à l'étape a) se compose de préférence de fibroblastes, dont des fibroblastes de souris établies en lignée. Parmi ces fibroblastes on trouve notamment les cellules STO qui peuvent ou non être modifiées ou transfectées par des vecteurs d'expression (Pain et al, 1996). Dans ce procédé, les cellules utilisées à l'étape a) sont des cellules obtenues par la mise en suspension de cellules provenant de disques blastodermiques d'oeufs fécondés dans un milieu de culture comprenant au moins une cytokine, b-FGF, et SCF. Lesdites cellules sont ensemencées sur un tapis de cellules nourricières, incubées, puis prélevées.

L'étape b) consiste en un sevrage progressif de chaque facteur de croissance ajouté dans le milieu de l'étape a), notamment une cytokine, b-FGF, et SCF, comprenant un passage dans un milieu neuf dépourvu d'au moins un desdits facteurs et à répéter différents passages successifs jusqu'à ce que le milieu soit dépourvu de l'ensemble desdits facteurs. Par sevrage progressif, on entend une suppression facteur par facteur dans le milieu de culture. Alternativement, on peut procéder à un sevrage drastique ou total, c'est-à-dire l'enlèvement de l'ensemble desdits facteurs en une seule fois. Ainsi, le sevrage à l'étape b) peut consister à diminuer la concentration d'un ou plusieurs facteurs progressivement ou à mettre en culture les cellules souches aviaires directement dans un milieu dépourvu d'un ou plusieurs facteurs ou encore dépourvu de la totalité desdits facteurs.

L'étape b) peut également comprendre le sevrage du sérum. A ce titre, le sevrage peut être progressif en diminuant la concentration en sérum lors de chaque passage, par exemple en passant de 10 % à 7,5% puis 3,75% et 2% pour tendre vers 0 % (milieu asérique). Alternativement, on peut procéder à un sevrage drastique.

L'étape b) peut également comprendre le sevrage du tapis nourricier. Le sevrage du tapis nourricier peut également être progressif en diminuant le nombre de cellules nourricières inactivées lors de chaque passage. Alternativement, on peut procéder à un sevrage drastique.

Bien entendu, l'ordre des sevrages peut varier. Par exemple, on peut commencer par le sevrage des facteurs de croissance et poursuivre avec le sevrage du tapis nourricier.

Ainsi, l'invention se rapporte aux lignées cellulaires établies et aux cellules issues desdites lignées susceptibles d'être obtenues à partir du procédé décrit ci-dessus, lesdites cellules étant capables de proliférer pendant au moins 50 jours, 100 jours, 150 jours, 300 jours, ou de préférence au moins 600 jours dans un milieu dépourvu de facteur de croissance exogène, de sérum et/ou de tapis nourricier.

Ces lignées cellulaires et les cellules qui en dérivent sont capables de proliférer pendant au moins 50 jours, 100 jours, 150 jours, 300 jours, ou de préférence au moins 600 jours dans un milieu de base, notamment dans un milieu tel que DMEM, GMEM, HamF12 ou McCoy supplémenté de différents additifs couramment utilisés par l'homme du métier. Parmi les additifs, on peut citer les acides aminés non essentiels, les vitamines et le pyruvate de sodium.
Ces cellules sont ainsi des lignées de cellules aviaires susceptibles d'être obtenue par un procédé comprenant les étapes de
a) mise en culture de cellules souches embryonnaires aviaires dans un milieu comportant :
   - au moins un facteur de croissance choisi parmi le SCF, IGF-1 et bFGF et au moins une cytokine choisie parmi le LIF, l'IL-11, l'IL-6, l'IL-6R, le CNTF, l'oncostatine et la cardiotrophine;
   - un tapis nourricier inactivé de cellules de fibroblastes de souris STO ; et
   - du sérum de veau foetal à une concentration de 12 à 8 %,
b) après une vingtaine de passages, modification du milieu de culture :
   - par sevrage progressif desdits facteurs de croissance et de ladite cytokine ; ou
   - par sevrage progressif du tapis nourricier ; ou
   - par diminution de la concentration en sérum de veau foetal jusqu'à arriver à des faibles proportions de 2 %,
c) établissement de lignées cellulaires adhérentes ou non-adhérentes capables de proliférer dans un milieu de base en l'absence de facteurs de croissance exogènes et de cytokine, de sérum ou du tapis nourricier inactivé, l'établissement de lignées cellulaires non-adhérentes étant obtenu par ensemencement par au moins 1x 10⁶ cellules/ml en boîte bactériologique suivi de plusieurs passages en dilution simple,
lesdites cellules de ladite lignée étant capables de proliférer pendant au moins 50 jours en culture *in vitro* dans un milieu de culture de base dépourvu de facteurs de croissance et de cytokines exogènes et présentant une réactivité avec des anticorps spécifiques sélectionnés dans le groupe SSEA-1 (TEC101) et EMA-1.

L'invention porte également sur les lignées cellulaires et les cellules issues de telles lignées décrites ci-dessus, caractérisées en ce qu'elles sont des cellules souches aviaires, en particulier des cellules souches embryonnaires aviaires.

Ces cellules souches peuvent être adhérentes tout en proliférant en l'absence du tapis nourricier inactivé. Alternativement, ces cellules souches sont non adhérentes et prolifèrent en suspension dans un milieu de base évoqué ci-dessus.

Ces cellules se caractérisent également en ce qu'elles présentent au moins une des caractéristiques suivantes :
- un rapport nucléocytoplasmique important,
- une activité alcaline phosphatase endogène,
- une activité télomérase endogène,
- une réactivité avec des anticorps spécifiques sélectionnés parmi le groupe des anticorps SSEA-1 (TEC01), SSEA-3, et EMA-1.

Avantageusement, ces cellules sont modifiées génétiquement de sorte à produire une substance d'intérêt, notamment un polypeptide d'intérêt, un anticorps ou un virus atténué.

Lesdites cellules peuvent par exemple supporter la réplication de virus vivants ou atténués, en particulier les virus sélectionnés dans le groupe des adénovirus, des hepadnavirus, des herpesvirus, des orthomyxovirus, des papovavirus, des paramyxovirus, des picornavirus, des poxvirus, des reovirus et des rétrovirus. De préférence, les virus répliqués sur ces cellules appartiennent à la famille des orthomyxovirus, en particulier le virus de la grippe ou à la famille des paramyxovirus, en particulier les virus de la rougeole, des oreillons et de la rubéole.

Ainsi, l'invention porte sur les lignées cellulaires décrites ci-dessus. De préférence, l'invention vise les lignées cellulaires issues de l'étape c) du procédé décrit plus haut caractérisées en ce qu'elles sont des cellules souches aviaires capables de croître indéfiniment en milieu de base dépourvu de facteurs de croissance exogènes, appauvri en sérum ou dépourvu de sérum et/ou de tapis nourricier.

Dans un autre aspect de l'invention, les cellules obtenues en fin d'étape c) peuvent être génétiquement modifiées.

La demande décrit également une culture cellulaire comprenant des cellules issues des lignées cellulaires décrites ci-dessus, notamment des cellules souches aviaires ou des cellules souches embryonnaires aviaires et un milieu de base dépourvu de facteurs de croissance exogènes, appauvri en sérum ou dépourvu de sérum et/ou du tapis nourricier inactivé.

Dans un aspect supplémentaire, l'invention concerne des lignées cellulaires caractérisée en ce que lesdites cellules répliquent de virus vivants ou atténués, en particulier les virus pris dans le groupe des adénovirus, des hepadnavirus, des herpesvirus, des orthomyxovirus, des papovavirus, des paramyxovirus, des picornavirus, des poxvirus, des reovirus et des rétrovirus.

De préférence, on utilise les lignées cellulaires et les cellules décrites précédemment pour la production de virus appartenant à la famille des orthomyxovirus, en particulier le virus de la grippe et pour la production de virus appartenant à la famille des paramyxovirus, en particulier les virus de la rougeole, des oreillons et de la rubéole.

Dans ces lignées et cellules, utilisées pour supporter la réplication de virus vivants ou atténués, on peut introduire le ou les composants nécessaires à l'accomplissement du cycle viral complet du virus de sorte à obtenir par exemple la sur-expression du récepteur au virus à la surface de la cellule.

Pour la suite de la description, on se référera aux légendes des figures ci-dessous.

### Légendes

**Figures 1-3** : **Courbes de croissance des lignées cellulaires de l'invention** (avec sevrage du sérum (figure 2) et avec sevrage du tapis nourricier (figure 3).
**Figure 4** : **Photo présentant la morphologie caractéristique des cellules souches aviaires**
   N : noyau, n : nucléole et C : cytoplasme
   (isolat S86N 99, grossissement X40, photo réalisée avec un appareil numérique Cyber-shot Sony).
**Figures 5A-5B** : **Photo présentant l'activité alcaline phosphatase des lignées de cellules souches aviaires adhérentes ou en suspension**
   Après fixation (0,1% formaldéhyde/0,5% glutaraldéhyde, 30 minutes à 4°C) les cellules sont rincées deux fois en PBS 1X et incubées entre 10 à 30 minutes à 37°C dans une solution de NBT/BCIP (Nitro Blue Tetrazolium chloride 0,375 mg/ml, 5-Bromo-4-Chloro-3-indolyl Phosphate 0,188 mg/ml, 0,1 M Tris pH 9.5, 0,05 M MgCl², 0,1M Nacl). La réaction est arrêtée par deux lavages PBS 1X et les photos sont réalisées.

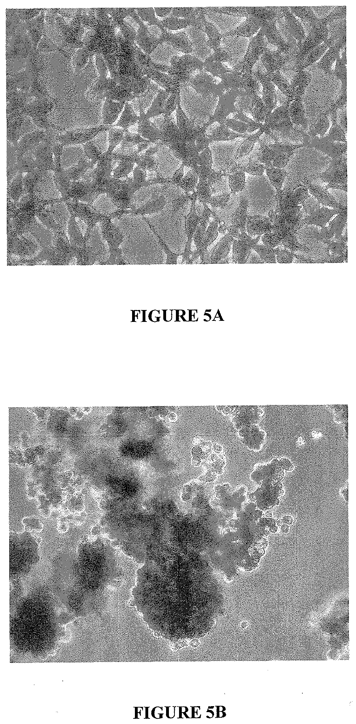

**Figure 5A** **:** illustre la coloration violette caractéristique de l'activité alcaline phosphatase endogène obtenue pour la lignée adhérente S86N45 p87, lignée cultivée sans feeder ni facteur (grossissement X40, appareil numérique Cyber-shot de Sony).
**Figure 5B** **:** illustre la coloration violette caractéristique de l'activité alcaline phosphatase endogène obtenue pour la lignée EB14 maintenue depuis 8 passages en suspension, lignée dérivée des cellules S86N 45, cultivée en suspension sans feeder ni facteur (grossissement X20, appareil numérique Cyber-shot de Sony).

### Exemple 1 : origine variable du matériel vivant utilisé

L'établissement de lignées cellulaires est souvent très lié à la nature génétique du matériel cellulaire. Ainsi, dans l'exemple murin, proportionnellement peu de fonds génétiques sont permissifs à l'obtention de cellules souches embryonnaires ES et implique souvent une notion d'animaux consanguins. Au niveau aviaire, il est difficile d'obtenir des animaux consanguins pour des raisons historiques et d'origine de sélection des souches commerciales ; l'intérêt étant d'éviter justement la consanguinité. Les oeufs sont la source initiale des cellules mises en culture dans cette invention. Les oeufs sont utilisés de préférence non incubés, mais quelques heures d'incubation peuvent être nécessaires pour obtenir les premiers stades de développement de l'embryon. Les cellules obtenues sont issues de différentes souches de poulet. Parmi les souches utilisées, on peut citer la souche S86N, souche commerciale destinée à la production de poulet Label, les CNR, souche destinée à la production de poulet Label, les Marens, souche locale bien caractérisée génétiquement et phénotypiqement, les White Leghorn, souche plus destinée à la production d'oeufs de consommation et souche de référence des laboratoires de recherche, etc. Dans cette dernière souche, différentes origines ont été testées dont certains oeufs (appelés Valo) issus de la souche White Leghorn de chez Lohmann (Allemagne) considérés comme des oeufs 'SPF' (SPF Specific Pathogen Free) maintenus dans des conditions très particulières de sécurité sanitaire. De nombreux isolats de cellules ont été obtenus à partir de différentes souches, suggérant le caractère général de la méthode.

### Exemple 2 : Obtention et établissement des cellules adhérentes

Les oeufs sont ouverts, le jaune est séparé du blanc lors de l'ouverture. Les embryons sont prélevés sur le jaune soit directement à l'aide d'une pipette Pasteur, soit à l'aide d'un petit filtre de papier absorbant (Papier Whatmann 3M), préalablement découpé sous forme d'un anneau perforé à l'aide d'une perforeuse. Le diamètre de la perforation étant d'environ 5 mm. Ces petits anneaux sont stérilisés à la chaleur sèche pendant environ 30 minutes au four. Ce petit anneau de papier est déposé sur la surface du jaune et centré au niveau de l'embryon qui est ainsi entouré par l'anneau de papier. Ce dernier est alors découpé à l'aide de petits ciseaux et l'ensemble prélevé est placé dans une boîte de Petri, remplie de PBS ou d'un liquide physiologique. L'embryon ainsi entraîné par l'anneau est nettoyé de l'excès de jaune dans le milieu et le disque embryonnaire, ainsi débarrassé de l'excès du vitellus, est prélevé avec une pipette Pasteur.

Dans les deux cas, les embryons sont mis dans un tube contenant du milieu physiologique (PBSIX, Tris Glucose, milieu, ...). Les embryons sont alors dissociés mécaniquement et ensemencés sur un 'feeder' dans du milieu de culture défini. Parmi les conditions préférentielles utilisées pour les mises en culture, on privilégie le milieu de culture composé du milieu MacCoy comme milieu de base supplémenté avec du sérum de veau foetal à une concentration initiale de 12 à 8 %, avec des acides aminés non essentiels à 1 %, avec un mélange de vitamines d'origine commerciale à 1 %, avec du pyruvate de sodium à une concentration finale de 1 mM, avec du beta-mercaptoéthanol à une concentration finale de 0,2 mM, de la glutamine à une concentration finale de 2,9 mM, avec un mélange initial d'antibiotiques contenant de la gentamycine à une concentration finale de 10 ng/ml, de la pénicilline à une concentration finale de 100 U/ml et de la streptomycine à une concentration finale de 100 µg/ml. Rapidement après les premiers passages des cellules, le mélange d'antibiotiques n'est plus ajouté dans le milieu. Par rapidement, on entend après les 3 à 5 premiers passages en général. Un mélange de nucléosides peut être ajouté également, ce mélange étant constitué comme décrit précédemment (Pain et al., 1996). Parmi les milieux de bases testés dans ces mêmes conditions et qui donnent des résultats similaires, on trouve les milieux Ham F12, Glasgow MEM et le DMEM, ce dernier supplémenté en biotine à une concentration finale de 8 mg/l. A titre de comparaison, la concentration de biotine est de 0,2 mg/l dans le milieu MacCoy, de 0, 0073 mg/l dans le Ham F12 et de 0 dans les milieux DMEM et GMEM commerciaux.

Les facteurs de croissance et les cytokines ajoutés dans le milieu de culture sont de façon privilégiée des facteurs et des cytokines recombinantes dont le SCF de souris à une concentration finale de 1 ng/ml, l'IGF-1 à une concentration finale de 1 à 5 ng/ml, le CNTF à une concentration finale de 1 ng/ml, l'IL-6 à une concentration finale de 1 ng/ml, et le récepteur soluble de l'IL-6 à une concentration finale de 0,5 ng/ml à 1 ng/ml. Dans certaines expériences, certains autres facteurs peuvent être ajoutés pendant les premiers passages. Par exemple jusqu'au passage 3 ou 10, on peut ajouter dans le milieu du bFGF à une concentration finale de 1 ng/ml et de l'IL-11 à une concentration finale de 1 ng/ml.

L'ensemencement est réalisé dans ce milieu sur le « feeder » inactivé composé de fibroblastes de souris établies en lignée, les cellules STO. Dans certains cas, ces cellules ont été transfectées avec des vecteurs d'expression simples permettant l'expression de facteurs de croissance comme le SCF aviaire, de façon constitutive dans les cellules STO. Ainsi, ce « feeder » produit le facteur sous une forme soluble et/ou attachée dans la membrane plasmique des cellules.

Après l'ensemencement initial des cellules directement dans ce milieu, le milieu est changé partiellement le lendemain, puis partiellement ou totalement durant les jours suivants, selon la vitesse d'adhésion observée des cellules primaires. Après environ 4 à 7 jours selon les cas, la culture initiale est dissociée et passée dans de nouvelles boîtes dans le même milieu initial sur le feeder inactivé. Après trois à cinq passages, les cellules sont cultivées sur un feeder inactivé de cellules STO non transfectées ou transfectées avec un vecteur d'expression codant pour une résistance à un antibiotique comme le gène de résistance à la néomycine, à l'hygromycine, à la puromycine, ..... Après une vingtaine de passages, les cellules sont progressivement sevrées en facteurs de croissance et cytokines. Par sevrage progressif, on entend une suppression facteur par facteur dans le milieu de culture. Ainsi à un passage, on enlève d'abord le SCF, puis, deux ou trois passages après, l'IGF-1. Si les cellules ne présentent pas d'altérations morphologiques ou de variation dans leur vitesse de prolifération moyenne, les autres facteurs, comme le CNTF, l'IL-6 sont ensuite supprimés. Ce sevrage peut être également drastique. On enlève alors en une seule fois tous les facteurs. Les cellules sont alors observées et ne sont passées que plusieurs jours après si leur vitesse de prolifération est modifiée. Cette dernière solution est en général celle qui est pratiquée.

Différents isolats sont ainsi obtenus et maintenus pendant des périodes de temps très longues. Par périodes de temps très longues, on entend des durées de l'ordre de plusieurs semaines avec un minimum de 50 jours, de préférence des durées supérieures à 200 à 400 jours, sans limitations dans le temps. Des durées supérieures à 600 jours sont observées.

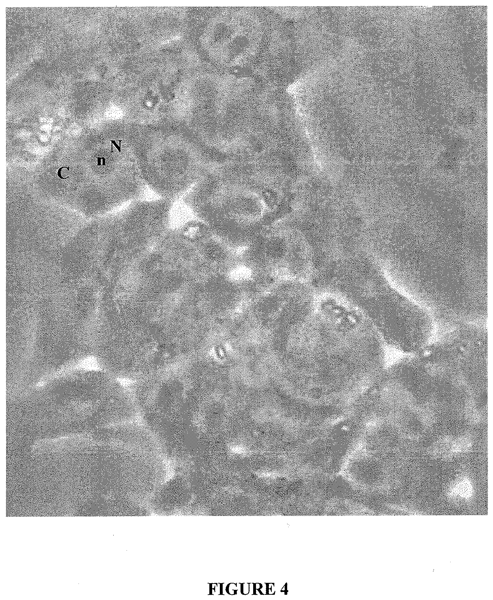

Quel que soit le support utilisé toutes les cellules qui sont adhérentes sont dissociées avec une enzyme protéolytique de dissociation, telle que la pronase, la collagénase, la dispase, la trypsine, etc. De préférence, un enzyme protéolytique d'origine bactérienne est utilisé, afin d'éviter tout contaminant potentiel d'origine animale. Ces cellules ont les caractéristiques des cellules souches embryonnaires avec une morphologie spécifique illustrée, à titre d'exemple, par la **photo de la** **Figure 4** i.e. une petite taille, un rapport nucléocytoplasmique important, un noyau avec au moins un nucléole bien visible et un très faible cytoplasme. Ces cellules sont caractérisées par une croissance en massifs plus ou moins compacts. Les cellules adhérentes et non adhérentes présentent une réactivité croisée avec un certain nombre d'anticorps, comme précédemment décrit dans Pain et al., 1996 et dans les brevets US 6,114,168 et EP 787 180. La composante activité télomérase endogène est également présente et est un élément important de la nature 'souche' de ces cellules.

Des cellules de différents isolats sont obtenues et maintenues pendant de longue période de temps.

**Le tableau 1** illustre quelques unes des caractéristiques de ces isolats.

| ***Nom*** | **Espèce** | **Début** | **« Arrêt »** | **jours** | **Passage** | **Génération** |
|---|---|---|---|---|---|---|
| | | | | | | |
| S86N 16 | Poulet S86N | 26-01-2000 | 05-08-2001 | 559 | 207 | 692 |
| WL3 | Poulet WL | 28-06-2000 | 09-08-2001 | 403 | 153 | 333 |
| Valo4 | Poulet Valo | 26-09-2000 | 07-02-2002 | 401 | 135 | 317 |
| S86N 45 | Poulet S86N | 29-01-2001 | 12-11-2001 | 287 | 118 | 329 |

On notera que le terme « arrêt » ne correspond pas à la fin de la prolifération des cellules mais à un arrêt volontaire des cultures de cellules de la part de l'expérimentateur. Le nombre de génération n est obtenu par la formule X= 2 n ou X est le nombre cumulé théorique de cellules. Ce nombre est disponible puisque les cellules sont comptées à chaque passage et lors de chaque ensemencement. L'historique complet de la culture est ainsi disponible.

### Exemple 3 : Passage des cellules

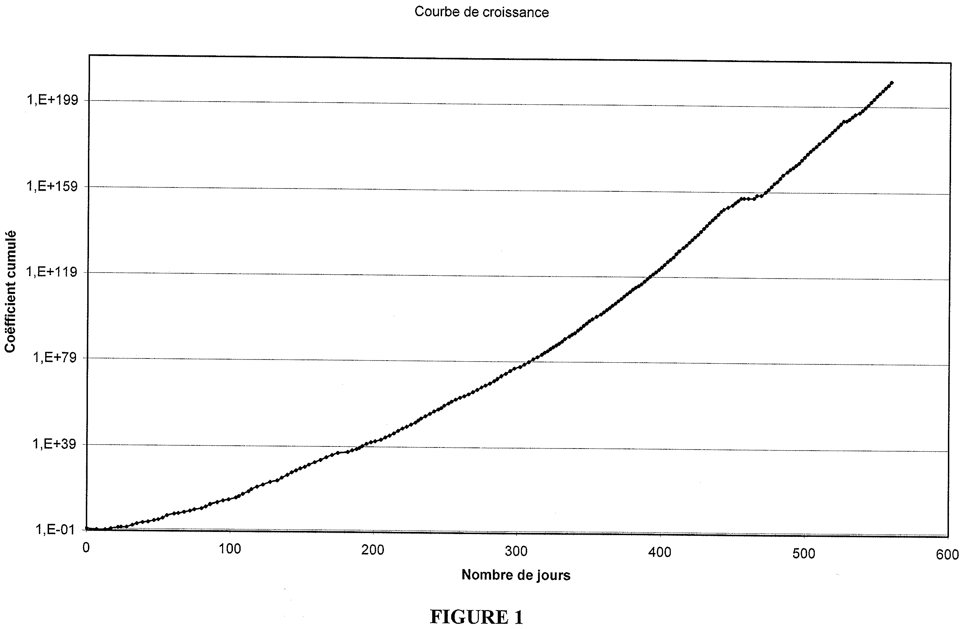

Une des caractéristiques des cellules souches, notamment des souches somatiques et des cellules souches embryonnaires, est leur aptitude à proliférer *in vitro* pendant des périodes de temps considérables. Afin de propager et de passer les cellules, le milieu de culture est changé et remplacé par du milieu neuf quelques heures avant leur passage. La courbe présentée en **figure 1** illustre un profil de croissance et d'établissement des cellules.

### Exemple 4 : temps de doublement et temps moyen de division

A partir des cellules établies en culture et présentées dans les exemples précédents, un temps moyen de division peut être calculé. Pour l'ensemble des isolats indépendants obtenus, la vitesse de prolifération s'accentue légèrement au cours des passages successifs faisant ainsi évoluer le temps moyen de division au cours de l'établissement des cellules. En phase adhérente, les cellules sont initialement ensemencées sur un tapis nourricier inactivé (appelé 'feeder') et sont passées régulièrement à une densité constante d'ensemencement initial de 1 à 2 x 10⁶ cellules par boîte de 100 mm. Le tableau 2 illustre le temps de doublement (d) et le temps moyen de division (TMD en heure) pour 3 types cellulaires établis en fonction du temps de culture. On observe que le temps moyen de doublement diminue au cours de l'établissement.

**Tableau 2 :**

| Cellules/Jours | 50 | 100 | 150 | 200 | 250 | 300 | 350 | 400 | 450 | 500 | 550 |
|---|---|---|---|---|---|---|---|---|---|---|---|
| | | | | | | | | | | | |
| S86N 16 (d) | 0,30 | 0,63 | 1,00 | 0,86 | 1,13 | 1,15 | 1,47 | 1,70 | 1,94 | 1,50 | 1,9 |
| S86N 16 (TMD) | 80 | 38 | 24 | 27,9 | 21,2 | 20,9 | 16,3 | 14,1 | 12,4 | 16 | 12,6 |
| S86N 45 (d) | 0,49 | 0,89 | 0,89 | 1,45 | 2,15 | x | x | x | x | x | x |
| S86N45 (TMD) | 49 | 26,8 | 27 | 16,5 | 11,1 | x | x | x | x | x | x |
| Valo 4 (d) | 0,03 | 0,61 | 1,00 | 1,17 | 1,26 | 1,03* | 1,08* | 1,25* | x | x | x |
| Valo 4 (TMD) | >48 | 39,3 | 24 | 20,5 | 19 | 23,3 | 22,2 | 19,2 | x | x | x |

Le temps de doublement moyen d est établi pour la période de temps indiquée en jours avec la formule suivante : d = (1/Log2 x (LogX2/X1)) x 1/(T2-T1) où X2 et X1 sont les nombres totaux de cellules aux instants T2 et T1. Cette formule est la conséquence directe du calcul du nombre de génération N par la formule X= 2ⁿ présenté dans l'exemple 1. Le Temps moyen de division (TMD) est alors obtenu en heures en divisant 24 heures par d.
* les cellules Valo sont passées au cours de cet établissement sur support plastique sans présence de feeder. Le temps de doublement diminue puis re-augmente, quand les cellules se réhabituent à ce nouvel environnement.

### Exemple 5 : Contrôle du niveau de sérum pour la prolifération des lignées

Lors de l'obtention de ces lignées, les milieux de culture utilisés sont des milieux de culture classiques comprenant une base (DMEM, GMEM, HamF12, McCoy, etc.) supplémentée de différents additifs tels que des acides aminés non essentiels, des vitamines, du pyruvate de sodium. Ce milieu complexe comprend du sérum de veau foetal, qui demeure un élément central de la culture, même si des composants de différentes origines dont des composants végétaux peuvent être progressivement utilisés. Un processus pour contrôler et habituer les cellules à des proportions relativement faibles de sérum de veau foetal est présenté. On parvient ainsi à maintenir des cellules en prolifération importante (Temps de division > 1) avec des pourcentages de sérum faible (2 % par exemple dans le cas des cellules S86N 16).

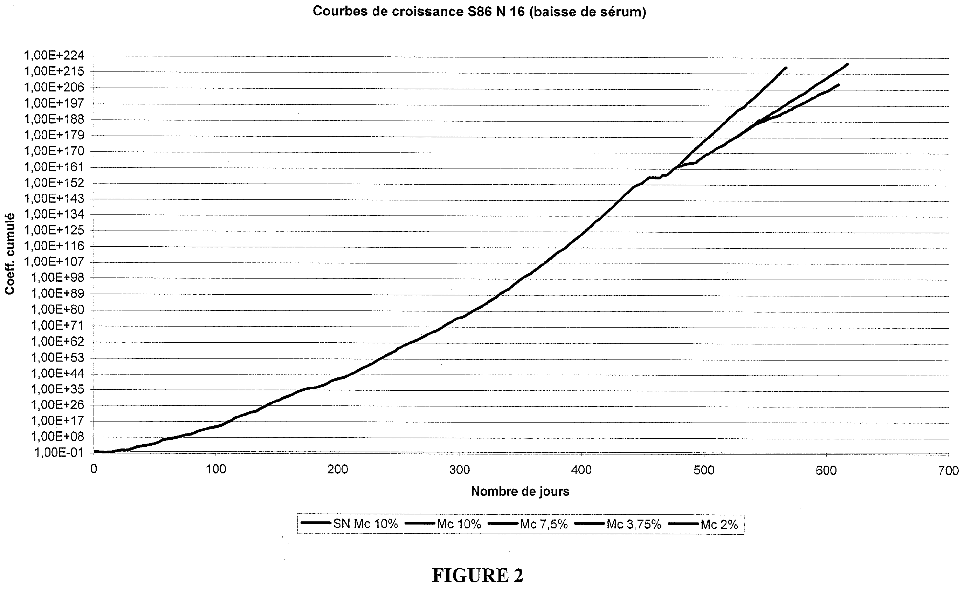

Les courbes présentées en **figure 2** illustrent la diminution relative de sérum pour un type cellulaire donné : les cellules S86N 16. Le temps de doublement et les temps moyens de division ont été également calculés et portés dans le **tableau 3.** On notera que le temps moyen de division augmente en fonction de la diminution relative de sérum. Une phase de rattrapage est néanmoins observée après quelques temps en culture dans les conditions mentionnées. Ce temps reste néanmoins inférieure à 24h (d>1), ce qui représente déjà une prolifération très intéressante en terme industriel même à des concentrations de sérum de 2 %, ce qui est déjà relativement faible. Des améliorations quant aux différents métabolites à utiliser peuvent être envisagées pour accroître ce temps et encore optimiser davantage les conditions de culture.

**Tableau 3 :**

| Condition | 10 % | 7,5 % | 3,75 % | 2 % |
|---|---|---|---|---|
| d | 2,02 | 1,51 | 1,47 | 1,08 |
| TMD | 11,9 | 15,8 | 16,3 | 22,2 |

Les exemples sont pris entre les passages p204 et p179 pour la condition 10 %, entre p198 et p176 pour le 7,5%, entre p224 et p201 pour le 3,75 % et entre p216 et p199 pour le 2 %.

### Exemple 6 : Sevrage des cellules en tapis nourricier

Dans les conditions initiales de culture, la présence d'un tapis de cellules inactivées semble nécessaire pour obtenir des cellules souches embryonnaires comme il a été décrit préalablement. Ce tapis nourricier ou 'feeder' ne semble plus nécessaire après un certain nombre de passages. Seul le plastique 'traité culture' apparaît important. En effet, une des caractéristiques de certaines cellules eucaryotes est de proliférer sous forme adhérente. Afin de faciliter l'adhésion des cellules, les différents matériels plastiques utilisés sont traités 'culture'. Ils subissent lors de leur fabrication un traitement qui ajoute des charges à la surface du plastique, charges qui favorisent les adhésions de la matrice extracellulaire des cellules. A l'inverse, le plastique non traité culture cellulaire, souvent appelé plastique de qualité bactériologique, n'est pas traité en surface par addition de charges spécifiques. L'adhésion des cellules y est en général très difficile, voir impossible, ou alors induit des changements de morphologie, de comportement souvent drastiques. Cette distinction entre les deux qualités de plastique permet d'obtenir selon les ensemencements qui y sont effectués des cellules avec des comportements différents. Un sevrage progressif des cultures en 'feeder' inactivé permet d'obtenir après quelques passages des cultures homogènes de cellules souches directement ensemencées sur plastique 'traité culture'.

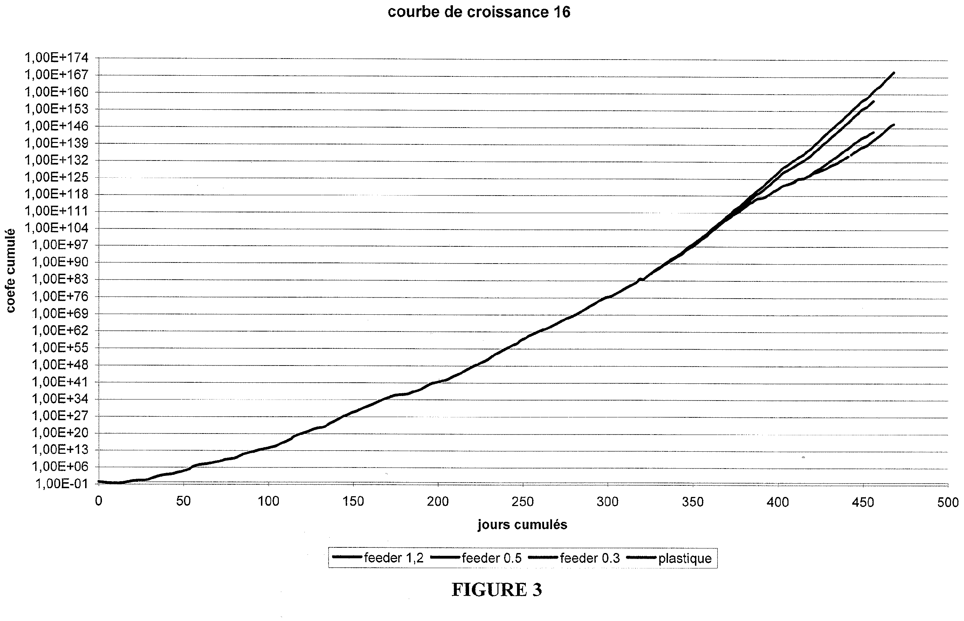

Les courbes de croissance comparative des cellules maintenues en présence et en absence de 'feeder' inactivé sont présentées avec le cas des cellules S86N 16 en **figure 3****.** Cette adaptation des cellules est progressive afin de ne pas perdre le caractère de cellules souches des cellules initialement maintenues sur 'feeder'. Des dérives progressives sont ainsi faites. L'obtention de cellules proliférant sur plastique est l'aboutissement du processus de sevrage. Dans le **tableau 4,** les temps de division montrent une sensibilité des cellules à leur environnement. Comme dans le cas du sevrage progressif en sérum, une adaptation est obtenue avec un effet de rattrapage sur les cellules après quelques passages dans les conditions définies.

**Tableau 4 :**

| Condition | 1,2 | 0,5 | 0,3 | plastique |
|---|---|---|---|---|
| d | 1,95 | 1,84 | 1,39 | 1,42 |
| TMD | 12,3 | 13 | 17,3 | 16,9 |

Les exemples sont pris entre les passages p154 et p131 pour les 3 conditions 1,2 x 10⁶, 0,5 x 10⁶ et 0,3 x 10⁶ cellules feeder et entre p161 et p139 pour la condition sur plastique seul.

### Exemple 7 : Sevrage des cellules en facteurs de croissance

Dans les conditions initiales de culture, la présence de facteurs de croissance est nécessaire. On peut distinguer schématiquement deux familles de facteurs : les cytokines et les facteurs trophiques.

Les cytokines sont principalement des cytokines dont l'action passe par un récepteur qui s'associe à la protéine gp130. Ainsi, le LIF, l'interleukine 11, l'interleukine 6, le CNTF, l'oncostatine, la cardiotrophine, ont un mode d'action semblable avec le recrutement au niveau du récepteur d'une chaîne spécifique et de l'association de cette dernière avec la protéine gp130 sous forme monomérique ou parfois hétérodimérique. Dans quelques cas, l'association d'une forme soluble des récepteurs, forme décrite entre autres pour les récepteurs de l'interleukine 6 et du CNTF, permet d'accroître l'effet prolifératif observé. Il a été montré préalablement que l'addition d'au moins une de ces cytokines semblait nécessaire à l'obtention des cellules souches embryonnaires.

Les facteurs trophiques sont principalement le SCF, l'IGF-1, le bFGF, qui sont également utilisés au départ de la culture, comme décrit précédemment. Leur présence est également nécessaire pour obtenir et amplifier les cellules.

En diminuant progressivement ces facteurs de croissance, il est possible d'obtenir après quelques passages des conditions de culture qui permettent la prolifération des cellules souches embryonnaires ou somatiques sans l'addition de facteur de croissance exogène. Les différents marqueurs utilisés pour caractériser ces cellules sont toujours positifs pour les cellules maintenues sans facteurs.

### Exemple 8 : Comparaison des milieux utilisés

Ensemencées dans des milieux différents, les cellules ne sont pas obtenues avec les mêmes fréquences. La comparaison des compositions des milieux rend difficile l'identification d'un des composants en particulier. Il semble plus vraisemblable que l'ensemble de l'association permette un mieux dans la physiologie des cellules. Parmi les milieux privilégiés, on notera le milieu Ham F12, MacCoy, DMEM et un milieu DMEM enrichi en biotine. A partir d'un même isolat, des essais d'adaptation dans ces différents milieux sont réalisés.

### Exemple 9 : Etablissement des cellules non adhérentes

Lors des passages successifs des cellules souches, un ensemencement à forte densité directement en boîte bactériologique permet d'obtenir après quelques passages des cellules embryonnaires qui se détachent de leur substrat et qui prolifèrent en suspension sous forme de petits agrégats réguliers. Cette prolifération est encouragée sur plusieurs passages par simple dilution, dissociation mécanique et non-usage d'enzyme protéolytique. L'agitation des cultures est en général pratiquée mais ne représente pas un élément discriminant pour l'obtention des cellules non adhérentes. Comme les cellules adhérentes, ces cellules présentent une morphologie caractéristique de cellules souches i.e. une petite taille, un rapport nucléocytoplasmique important, un noyau avec au moins un nucléole bien visible et un très faible cytoplasme. Ces cellules sont caractérisées par une croissance en petits agrégats plus ou moins compacts. Ces cellules non adhérentes présentent une réactivité croisée avec un certain nombre d'anticorps, comme précédemment décrit dans Pain et al., 1996. Ces cellules sont également positives pour l'activité télomérase endogène (comme présenté dans l'exemple 10 pour les cellules EB1, EB4 et EB5). En phase non adhérente, les cellules présentent une prolifération importante dans différents milieux. La densité initiale d'ensemencement ainsi que l'approvisionnement en milieu frais de façon très régulière assure des densités importantes qui peuvent aller au-delà de 1 x 10⁶ cellules par ml. Le **tableau 5** résume les principales caractéristiques de quelques isolats (cellules parentales, passage initial de la mise en suspension, nombre de jours maintenus en culture en suspension, nombre de passages et de générations obtenues avant un arrêt volontaire des entretiens). On peut ainsi remarquer que le passage pour la mise en suspension peut varier d'un isolat à l'autre (voir isolat EB1 et EB14) ainsi que la vitesse de prolifération (voir isolat EB3 et EB14).

**Tableau 5 :**

| Nom | Cellules parentales | Passage initial | Début | jours | Passages | Générations |
|---|---|---|---|---|---|---|
| EB1 | S86N 16 | p111 | 20-01-2001 | 184 | 41 | 120 |
| EB3 | S86N 16 | p118 | 23-01-2001 | 381 | 17 | 40 |
| EB4 | S86N 45 | p100 | 25-09-2001 | 44 | 17 | 40 |
| EB5 | S86N 45 | p100 | 25-09-2001 | 44 | 17 | 40 |
| EB14 | S86N 45 | p81 | 5-09-2002 | 70 | 24 | 65 |

On notera que le terme « Début » correspond à la mise en non-adhérence des cellules.

### Exemple 10 : Caractérisation des cellules établies

Les cellules souches maintenues pendant des temps longs de culture sont caractérisées avec les mêmes critères que ceux décrits précédemment (Pain et al., 1996). On peut ainsi détecter de façon régulière l'activité alcaline phosphatase endogène, illustrée par la **photo de la** **figure 5****,** l'activité télomérase endogène et la réactivité avec certains anticorps spécifiques comme les anticorps SSEA-1 (TEC-01) et EMA-1.

Un des critères importants lors de l'établissement des cellules est la présence de la télomérase. Différents tests ont été faits au cours du maintien en culture des cellules à l'aide d'un kit de détection de la TRAP (Telomerase PCR Elisa, Roche). Les cellules sont détectées positives après différents passages en culture. Ainsi, l'activité télomérase est détectable pour les cellules S86N 16, les cellules S86N 45 ainsi que pour les cellules EB1, EB4 et EB5 qui en sont dérivées sous une forme non adhérente (voir **tableau 6**). Les CEF (Chicken Embryonic Fibroblasts) maintenus en culture primaire sont considérés comme négatifs. Le seuil d'une D.O. < 0,2 est le seuil recommandé par le kit comme le seuil négatif. Toutes les analyses ont été réalisées sur un équivalent de 2000 cellules.

**Tableau 6 : Dosage de l'activité télomérase dans différentes lignées à différents passages**

| Cellules | Passage | Télomérase D.O. |
|---|---|---|
| S86N 16 | p12 | 1,7 |
| | p29 | 2,8 |
| | p185 | 0,97 |
| | p204 | 0,95 |
| S86N 16 EB1 | p134 | 1,1 |
| S86N 45 | p50 | 0,87 |
| | p58 | 1,1 |
| | p66 | 0,96 |
| | p94 | 1,2 |
| S86N 45 EB 4 | p112 | 1,4 |
| S86N 45 EB5 | p112 | 0,94 |
| CEF * | p4 | 0,07 |

### Exemple 11 : Transfection et induction des cellules

Les cellules souches maintenues en croissance sur le long terme sont transfectées avec différents plasmides d'expression. Il a été montré que les cellules souches aviaires pouvaient être transfectées (Pain et al., 1999). En particulier, les cellules non adhérentes sont transfectées et différents systèmes de tri permettent de repérer les cellules transfectées de façon stable (tri cellulaire, dilution limite, ...). Ces modifications génétiques peuvent s'effectuer au stade non différencié de la cellule souche. Cette modification obtenue, la cellule est alors induite à différencier spontanément ou par addition d'inducteur de différenciation. Dans ce cas, on peut utiliser l'acide rétinoïque à des concentrations de 10⁻⁸ M à 10⁻⁶ M, ou le diméthysulfoxide à des concentrations de 1 à 2 % final ou le butyrate de sodium à des concentrations de 10⁻⁴ à 10⁻⁸ M, ou le phorbol ester (TPA, PMA, ...) ou encore les lipopolysaccharrides (LPS) à des concentrations de 1 à 5 µg /ml final. Dans un autre exemple, les cellules peuvent former des corps embryoïdes en suspension, corps embryoïdes qui peuvent être mis à adhérer sur plastique après dissociation ou non des cellules qui les constituent. Ces cellules différenciées prolifèrent alors mais ont une capacité plus limitée de prolifération dans le long terme. En ciblant la modification génétique sur un gène qui influence la prolifération des cellules, on peut rendre ces cellules différenciées capables de proliférer sur le long terme.

### Exemple 12 : Infection des cellules

Les cellules adhérentes et non adhérentes sont infectables par différents virus et rétrovirus dont les virus et rétrovirus aviaires. Ces cellules peuvent ainsi servir de support de réplication pour la production de stocks viraux destinés à la production de vaccins humains et vétérinaires vivants, atténués ou inactivés selon les cas. Parmi les virus d'intérêt, on peut citer ceux de la famille des adénovirus (comme Human Adenovirus C, Fowl Adenovirus A, Ovine adenovirus D, Turkey Adenovirus B), des circoviridés (comme Chicken Anemia Virus, CAV), certains coronavirus, comme le virus de la bronchite infectieuse aviaire (IBV), les flavivirus (comme Yellow fever virus et hepatitis C virus), les hepadnavirus (comme Hepatitis B virus et Avihepadnavirus tels que Duck hepatitis B virus); les herpesvirus (comme les Gallid herpesvirus, HSV (Herpes simplex virus) et Human herpesvirus 1, 3 et 5), les orthomyxovirus (comme le virus de la grippe : Influenzavirus A, Influenzavirus B et Influenzavirus C), les papovavirus (comme polyomavirus et plus particulièrement Simian virus 40), les paramyxovirus (comme les virus de la rougeole, des oreillons, de la rubéole et comme les respirovirus et pneumovirus tels que Human respiratory syncytial virus et Metapneumovirus tels que Avian pneumovirus), les picornavirus (comme le virus de la polio, de l'hépatite A, et tel que Encephalomyocarditis virus et Foot-and-mouth disease virus), les poxvirus, (comme les fowlpox virus et les avipox dont les Canarypox les Juncopox virus, les Mynahpox virus, les Pigeonpox virus, les Psittacinepox virus, les Quailpox virus, les Sparrowpox virus, les Starlingpox virus, les Turkeypox virus), les reovirus (comme les rotavirus), les rétrovirus (comme les ALV, avian leukosis virus, les Gammaretrovirus tels que Murine leukeamia virus, les Lentivirus tel que Human immunodeficiency virus 1 et 2) et les Togaviridae tels que Rubivirus, notamment Rubella virus.

### Exemple 13 : Protocole d'infection d'une lignée cellulaire aviaire non adhérente (EB1) par un virus

### Amplification des cellules :

Les cellules EB1 ou EB14 sont ensemencées dans un milieu, de préférence du MacCoy's 5A, du HAMF12, du DMEM, ou tout autre milieu intéressant, contenant 5 % de sérum à une concentration de 0,2 10⁶ cellules/ml pour un volume initial de 50 ml en général. Elles sont maintenues en culture à 39°C et à 7,5 % de CO₂, sous agitation. Du milieu neuf est ajouté chaque jour pendant les 3 à 4 jours que durent l'amplification pour atteindre une concentration cellulaire de 1 à 3 x 10⁶ cellules/ml pour un volume final de culture de 100 à 250 ml.

Les cellules en suspension sont prélevées et centrifugées pendant 10 min à 1000 rpm environ. Le culot est resuspendu dans 20 à 50 ml de PBS 1X (Phosphate buffer Salt). Les cellules sont alors comptées, centrifugées et les cellules en culot sont reprises dans un milieu sans sérum à une concentration finale de 3 à 5,10⁶ cellules/ml. Plusieurs tubes sont alors préparés dans ces conditions contenant de 3 à 5 x 10⁶ cellules par tube.

### Préparation du virus et infection :

Le stock viral de titre connu est décongelé rapidement à 37°C et dilué dans du milieu sans sérum à un titre de 10 x à 1000 x la concentration nécessaire à l'infection finale. Les cellules sont infectées par le virus d'intérêt à une m.o.i. (multiplicity of infection) de 0,01 à 0,5 selon les types de virus, ce qui fait ajouter entre 0,1 et 10 % volume/volume de suspension virale au culot cellulaire. Après 1 heure d'incubation à la température optimale pour le virus, en général de 33 à 37°C, les cellules sont centrifugées à nouveau et le milieu enlevé avec précaution. Cette étape s'avère souvent nécessaire pour limiter l'effet du virus initial dans le processus ultérieur. Une des possibilités est de diluer directement les cellules sans les centrifuger à nouveau avec du milieu avec sérum (5 % de sérum) à une concentration finale de 0,2 à 1.10⁶ cellules/ml et remises en incubation.

### Récolte du surnageant et des cellules :

Après 2 à 4 jours d'incubation, selon les cinétiques virales et l'effet cytopathique éventuel de certains virus, le milieu contenant les cellules ou les débris cellulaires est récolté. Selon les virus, seuls le culot ou le surnageant peuvent être intéressants et contenir les particules virales. Les cellules sont récoltées et centrifugées. Le surnageant recueilli est centrifugé de nouveau 5 à 10 minutes à 2500 rpm, et conservé à -80°C avant la purification des particules. Un aliquot est prélevé pour la réalisation du titrage. Le culot cellulaire est repris dans 5 ml de milieu sans sérum, soniqué, et centrifugé 5 à 10 minutes à 2500 rpm. Le surnageant obtenu est conservé à -80°C jusqu'à la purification et le titrage d'un aliquot.

Les efficacités d'infection et de production virales sont comparées entre les différentes conditions réalisées.

Pour les virus à effets cytopathiques, les titrages sont en général réalisés par la technique des plages de lyse.

### Exemple 14 : Protocole d'infection d'une lignée cellulaire aviaire adhérente (S86N 45) par un virus

### Préparation des cellules :

Les cellules sont ensemencées 48 heures avant l'infection dans des flacons T150 à une concentration comprise entre 0,03 et 0,06 10⁶ cellules/cm² dans un milieu, de préférence du milieu MacCoy's 5A, du HAMF12, du DMEM, ou tout autre milieu intéressant, contenant 5 % de sérum. Elles sont maintenues à 39°C et 7,5 % CO₂.

### Infection :

Le stock viral de titre connu est décongelé rapidement à 37°C et dilué dans du milieu sans sérum à un titre de 10 x à 1000 x la concentration nécessaire à l'infection finale. Les cellules sont infectées par le virus d'intérêt à une m.o.i. (multiplicity of infection) de 0,01 à 0,5 selon les types de virus, ce qui fait ajouter entre 0,1 et 10 % volume/volume de suspension virale au culot cellulaire. L'infection est en général réalisée dans un minimum de milieu (de 5 à 10 ml pour un flacon de 75 cm²) dans un milieu à 0 % sérum.

Après 1 heure d'incubation à la température optimale pour le virus, en général de 33 à 37°C, 20 ml de milieu 5 % sont rajoutés dans les flacons. Dans un cas particulier, les cellules peuvent être lavées avec du PBS pour éliminer les particules qui ne seraient pas attachées aux cellules. Dans le cas d'un virus cytopathique, les cellules sont observées quotidiennement après l'infection pour suivre l'apparition de plage de lyse cellulaire, indicatrice du bon déroulement de l'infection.

### Récolte du surnageant et des cellules :

Après 2 à 4 jours d'incubation selon les cinétiques virales et l'effet cytopathique éventuel de certains virus, le milieu contenant le surnageant, les cellules et les débris cellulaires sont récoltés. Selon les virus, seuls le culot ou le surnageant peuvent être intéressants et contenir les particules virales. Les cellules sont récoltées et centrifugées. Le surnageant recueilli est centrifugé de nouveau 5 à 10 minutes à 2500 rpm, et conservé à -80°C avant la purification des particules. Un aliquot est prélevé pour la réalisation du titrage. Le culot cellulaire est repris dans 5 ml de milieu sans sérum, soniqué, et centrifugé 5 à 10 minutes à 2500 rpm. Le surnageant obtenu est conservé à -80°C jusqu'à la purification et le titrage d'un aliquot.

Les efficacités d'infection et de production virales sont comparées entre les différentes conditions réalisées.

Pour les virus à effet cytopathique, les titrages sont en général réalisés par la technique des plages de lyse.

### Exemple 15 : Réplication d'un avipox recombinant sur les cellules souches aviaires non adhérentes de la lignée EB1

Les cellules souches non adhérentes EB1 à passage138 sont amplifiées puis infectées à une m.o.i de 0,1 par un avipox recombinant produisant une protéine d'intérêt.

Après l'infection, les cellules sont maintenues en spinner pendant les 4 jours que dure l'infection. Un aliquot est prélevé à partir du deuxième jour puis les deux jours suivants pour suivre l'évolution du titre viral à la fois au niveau du surnageant et au niveau du contenu intracellulaire, après lyse du contenu cellulaire. La titration a été réalisée par la technique des plages de lyse.

Le **tableau 7** illustre les résultats obtenus. Ces résultats mettent en évidence la réplication très satisfaisante de l'avipox recombinant sur les cellules souches EB 1. Ainsi le titre infectieux progresse tout au long de la culture et du déroulement de l'infection pour atteindre un maximum après 4 jours d'incubation de 7.2 PFU/cellule (PFU : Plating Forming Unit). Ce titre est au moins équivalent à celui obtenu pour ce même avipox recombinant sur des cellules primaires d'embryons de poulet.

Ce titre est susceptible d'être amélioré par des conditions particulières de culture et des processus d'optimisation.

On notera également que des titres infectieux au moins équivalents ont également été obtenus à plus grande échelle dans des bioréacteurs de 3 litres.

**Tableau 7 : Cinétique de titrage de l'avipox recombinant sur les cellules souches EB1 non adhérentes**

| Prélèvement (h après infection) | **50 heures** | **74 heures** | **97 heures** |
|---|---|---|---|
| **fraction cellulaire** (Log PFU/ml) | 6,40 | 6,37 | 5,99 |
| **Surnageant** (Log PFU/ml) | 5,56 | 5,8 | 6,29 |
| **Total** (Log PFU/ml) | 5,78 | 5,94 | 6,31 |
| **PFU/cellule** | **2,2** | **3,2** | **7,2** |

### REFERENCES

Baba TW, Humphries EH. (1985). Formation of a transformed follicle is necessary but not sufficient for development of an avian leukosis virus-induced lymphoma. Proc Natl Acad Sci USA, 82:213-216.
Beug H, von Kirchbach A, Doderlein G, Conscience JF, Graf T. (1979). Chicken hematopoietic cells transformed by seven strains of defective avian leukemia viruses display three distinct phenotypes of differentiation. Cell 18:375-390.
Guilhot C, Benchaibi M, Flechon JE, Samarut J. (1993). The 12S adenoviral E1A protein immortalizes avian cells and interacts with the avian RB product. Oncogene 8:619-624.
Kawaguchi T, Nomura K, Hirayama Y, Kitagawa T. (1987). Establishment and characterization of a chicken hepatocellular carcinoma cell line, LMH. Cancer Res., 47:4460-4464.
Kim H, You S, Farris J, Foster LK, Foster DN. (2001). Post-transcriptional inactivation of p53 in immortalized chicken embryo fibroblast cells. Oncogene 20:3306-3310.
Kim H, You S, Kim IJ, Foster LK, Farris J, Ambady S, Ponce de Leon FA, Foster DN. (2001). Alterations in p53 and E2F-1 function common to immortalized chicken embryo fibroblasts. Oncogene 20:2671-2682.
Liu JL, Klein PA, Moscovici MG, Moscovici C. (1992). Monoclonal antibodies recognizing normal and retrovirus-transformed chicken hematopoietic cells. Virology 189:583-591.
Moscovici C, Moscovici MG, Jimenez H, Lai MM, Hayman MJ, Vogt PK. (1977). Continuous tissue culture cell lines derived from chemically induced tumors of Japanese quail. Cell 11:95-103.
Pain B., Clark M.E., Shen M., Nakazawa H., Sakurai M., Samarut J., Etches RJ (1996). Long-term in vitro culture and characterisation of avian embryonic stem cells with multiple morphogenetic potentialities. Development 122:2339-2348.
Pain B., Chenevier P., Samarut J. (1999). Chicken embryonic stem cells and transgenic strategies. Cells Tissues Organs 165:212-219.
Samarut J, Gazzolo L. (1982). Target cells infected by avian erythroblastosis virus differentiate and become transformed. Cell 28:921-929.
Smith JR and Pereira-Smith OM (1996). Replicative senescence: implications for in vivo aging and tumor suppression. Science 273, 63-67.

## Revendications

1. Lignée de cellules aviaires susceptible d'être obtenue par un procédé comprenant les étapes de
a) mise en culture de cellules souches embryonnaires aviaires dans un milieu comportant :
- au moins un facteur de croissance choisi parmi le SCF, IGF-1 et bFGF et au moins une cytokine choisie parmi le LIF, l'IL-11, l'IL-6, l'IL-6R, le CNTF, l'oncostatine et la cardiotrophine;
- un tapis nourricier inactivé de cellules de fibroblastes de souris STO ; et
- du sérum de veau foetal à une concentration de 12 à 8 %,
b) après une vingtaine de passages, modification du milieu de culture :
- par sevrage progressif dudit ou desdits facteur(s) de croissance et de ladite ou lesdites cytokine(s) ; ou
- par sevrage progressif du tapis nourricier ; ou
- par diminution de la concentration en sérum de veau foetal jusqu'à arriver à des faibles proportions de 2 %,
c) établissement de lignées cellulaires adhérentes ou non-adhérentes capables de proliférer dans un milieu de base en l'absence de facteurs de croissance exogènes et de cytokine, de sérum ou du tapis nourricier inactivé, l'établissement de lignées cellulaires non-adhérentes étant obtenu par ensemencement par au moins 1x 10⁶ cellules/ml en boîte bactériologique suivi de plusieurs passages en dilution simple,
lesdites cellules de ladite lignée étant capables de proliférer pendant au moins 50 jours en culture *in vitro* dans un milieu de culture de base dépourvu de facteurs de croissance et de cytokines exogènes et présentant une réactivité avec des anticorps spécifiques sélectionnés dans le groupe SSEA-1 (TEC01) et EMA-1.

2. Lignée de cellules souches aviaires selon la revendication 1, **caractérisée en ce que** ladite lignée présente au moins une des caractéristiques suivantes :
- un nucléole bien visible et un très faible nucléoplasme;
- une activité télomérase endogène ;
- et une activité alcaline phosphatase endogène.

3. Lignée de cellules selon l'une des revendications 1 ou 2, **caractérisée en ce que** l'étape b) comprend également un sevrage progressif du sérum de veau foetal, lesdites cellules de ladite lignées étant capables de proliférer en l'absence de sérum de veau foetal et de facteurs de croissance et de cytokines exogènes.

4. Lignée de cellules selon l'une des revendications 1 à 3, **caractérisée en ce que** l'étape b) comprend également un sevrage progressif du tapis nourricier, lesdites cellules de ladite lignées étant capables de proliférer en l'absence de tapis nourricier et de facteurs de croissance et de cytokines exogènes.

5. Lignée de cellules aviaires selon l'une des revendications 1 à 4, **caractérisée en ce que** les cellules de ladite lignée sont capables de proliférer pendant au moins 100 jours.

6. Lignée de cellules aviaires selon l'une des revendications 1 à 4, **caractérisée en ce que** les cellules de ladite lignée sont capables de proliférer pendant au moins 150 jours.

7. Lignée de cellules aviaires selon l'une des revendications 1 à 4, **caractérisée en ce que** les cellules de ladite lignée sont capables de proliférer pendant au moins 300 jours.

8. Lignée de cellules aviaires selon l'une des revendications 1 à 4, **caractérisée en ce que** les cellules de ladite lignée sont capables de proliférer pendant au moins 600 jours.

9. Lignée de cellules aviaires selon l'une des revendications 1 à 8, **caractérisée en ce que** ledit milieu de culture de base est choisi dans le groupe constitué des DMEM, GMEM, HamF12 et McCoy, supplémenté avec des acides aminés non essentiels, des vitamines et du pyruvate de sodium.

10. Lignée de cellules aviaires selon l'une des revendications 1 à 9, **caractérisée en ce que** ladite lignée cellulaire est une lignée de cellules adhérentes.

11. Lignée de cellules aviaires selon l'une des revendications 1 à 9, **caractérisée en ce que** ladite lignée cellullaire est une lignée de cellules non-adhérentes qui prolifèrent en suspension.

12. Lignée de cellules aviaires selon l'une des revendications 1 à 11, **caractérisée en ce que** lesdites cellules répliquent un virus vivant ou atténué, ledit virus étant choisi dans le groupe des adénovirus, hepadnavirus, des herpesvirus, des orthomyxovirus, des papovavirus, des paramyxovirus, des picomavirus, des poxvirus, des réovirus et des rétrovirus.

13. Lignée de cellules aviaires selon la revendication 12, **caractérisée en ce que** ledit virus est un orthomyxovirus et est le virus de la grippe.

14. Lignée de cellules aviaires selon la revendication 12, **caractérisée en ce que** ledit virus est un paramyxovirus sélectionné dans le groupe constitué des virus de la rougeole, des oreillons, et de la rubéole.

15. Lignée de cellules aviaires selon la revendication 12, **caractérisée en ce que** ledit virus est un poxvirus sélectionné dans le groupe constitué des canarypox virus et fowlpox virus.

## Patentansprüche

1. Vogelzelllinie, erhältlich durch ein Verfahren, umfassend die Schritte
a) Kultivieren von embryonalen Vogelstammzellen in einem Medium, umfassend:
- mindestens einen Wachstumsfaktor, ausgewählt aus SCF, IGF-1 und bFGF, und mindestens ein Cytokin, ausgewählt aus LIF, IL-11, IL-6, IL-6R, CNTF, Onkostatin und Cardiotrophin;
- eine inaktivierte Feeder-Schicht von STO-Maus-Fibroblastenzellen; und
- fötales Kälberserum in einer Konzentration von 12 bis 8%,
b) nach etwa zwanzig Passagen Modifizieren des Kulturmediums:
- durch fortschreitendes Entziehen des Wachstumsfaktors (der Wachstumsfaktoren) und des Cytokins (der Cytokine); oder
- durch fortschreitendes Entziehen der Feeder-Schicht; oder
- durch Verringern der Konzentration von fötalem Kälberserum bis zum Erreichen geringer Gehalte von 2%,
c) Etablieren adhärenter oder nicht-adhärenter Zelllinien, die zur Proliferation in einem Basismedium in Abwesenheit von exogenen Wachstumsfaktoren und Cytokin, Serum oder inaktivierter Feeder-Schicht im Stande sind, wobei das Etablieren nicht-adhärenter Zellinien erzielt wird durch Animpfen mit mindestens 1x10⁶ Zellen/ml in einer bakteriologischen Schale, gefolgt von mehreren Passagen in einfacher Verdünnung,
wobei die besagten Zellen der besagten Linie zum Proliferieren in *In-vitro*-Kultur in einem Basiskulturmedium, dem exogene Wachstumsfaktoren und Cytokine fehlen, für mindestens 50 Tage im Stande sind und eine Reaktivität mit spezifischen Antikörpern zeigen, die aus der Gruppe SSEA-1 (TEC01) und EMA-1 ausgewählt sind.

2. Linie von Vogelstammzellen gemäß Anspruch 1, **dadurch gekennzeichnet, dass** die Linie mindestens eine der folgenden Eigenschaften aufweist:
- ein deutlich sichtbarer Nucleolus und ein sehr schwaches Nucleoplasma;
- eine endogene Telomerase-Aktivität;
- und eine endogene Alkalische-Phosphatase-Aktivität.

3. Zelllinie gemäß einem der Ansprüche 1 oder 2, **dadurch gekennzeichnet, dass** der Schritt b) auch das fortschreitende Entziehen des fötalen Kälberserums umfasst, wobei die Zellen der besagten Linie zum Proliferieren in Abwesenheit von fötalem Kälberserum und exogenen Wachstumsfaktoren und Cytokinen im Stande sind.

4. Zelllinie gemäß einem der Ansprüche 1 bis 3, **dadurch gekennzeichnet, dass** der Schritt b) auch ein fortschreitendes Entziehen der Feeder-Schicht umfasst, wobei die Zellen der besagten Linie zum Proliferieren in Abwesenheit einer Feeder-Schicht und exogener Wachstumsfaktoren und Cytokinen im Stande sind.

5. Vogelzelllinie gemäß einem der Ansprüche 1 bis 4, **dadurch gekennzeichnet, dass** die Zellen der besagten Linie zum Proliferieren für mindestens 100 Tage im Stande sind.

6. Vogelzelllinie gemäß einem der Ansprüche 1 bis 4, **dadurch gekennzeichnet, dass** die Zellen der besagten Linie zum Proliferieren für mindestens 150 Tage im Stande sind.

7. Vogelzelllinie gemäß einem der Ansprüche 1 bis 4, **dadurch gekennzeichnet, dass** die Zellen der besagten Linie zum Proliferieren für mindestens 300 Tage im Stande sind.

8. Vogelzelllinie gemäß einem der Ansprüche 1 bis 4, **dadurch gekennzeichnet, dass** die Zellen der besagten Linie zum Proliferieren für mindestens 600 Tage im Stande sind.

9. Vogelzelllinie gemäß einem der Ansprüche 1 bis 8, **dadurch gekennzeichnet, dass** das Basiskulturmedium ausgewählt ist aus der Gruppe bestehend aus DMEM, GMEM, HamF12 und McCoy, ergänzt mit nicht-essentiellen Aminosäuren, Vitaminen und Natriumpyruvat.

10. Vogelzelllinie gemäß einem der Ansprüche 1 bis 9, **dadurch gekennzeichnet, dass** die Zelllinie eine Linie adhärenter Zellen ist.

11. Vogelzelllinie gemäß einem der Ansprüche 1 bis 9, **dadurch gekennzeichnet, dass** die Zelllinie eine Linie nicht-adhärenter Zellen ist, die in Suspension proliferieren.

12. Vogelzelllinie gemäß einem der Ansprüche 1 bis 11, **dadurch gekennzeichnet, dass** die Zellen ein lebendes oder abgeschwächtes Virus replizieren, wobei das Virus ausgewählt ist aus der Gruppe von Adenoviren, Hepadnaviren, Herpesviren, Orthomyxoviren, Papovaviren, Paramyxoviren, Picornaviren, Pockenviren, Reoviren und Retroviren.

13. Vogelzelllinie gemäß Anspruch 12, **dadurch gekennzeichnet, dass** das Virus ein Orthomyxovirus ist und das Grippevirus ist.

14. Vogelzelllinie gemäß Anspruch 12, **dadurch gekennzeichnet, dass** das Virus ein Paramyxovirus ist, ausgewählt aus der Gruppe bestehend aus Masern-, Mumps- und Rötelnviren.

15. Vogelzelllinie gemäß Anspruch 12, **dadurch gekennzeichnet, dass** das Virus ein Pockenvirus ist, ausgewählt aus der Gruppe bestehend aus Kanarienpockenvirus und Geflügelpockenvirus.

## Claims

1. Avian cell line obtainable by a process comprising the steps of
a) culturing avian embryonic stem cells in a medium comprising:
- at least one growth factor chosen from SCF, IGF-1 and bFGF and at least one cytokine chosen from LIF, IL-11, IL-6, IL-6R, CNTF, oncostatin and cardiotrophin;
- an inactivated feeder layer of mouse STO fibroblasts;
and
- foetal calf serum at a concentration of 12 to 8%;
b) after approximately twenty passes, modifying the culture medium:
- by gradually withdrawing said growth factor(s) and said cytokine(s); or
- by gradually withdrawing the feeder layer; or
- by decreasing the concentration of foetal calf serum until low concentrations of 2% are reached,
c) establishing adherent or nonadherent cell lines capable of proliferating in a basal medium in the absence of exogenous growth factors and cytokines, serum or inactivated feeder layer, the establishment of nonadherent cell lines being obtained by inoculation with at least 1 x 10⁶ cells/ml in a bacteriological dish followed by several passes of simple dilution,
said cells of said line being able to proliferate for at least 50 days in *in vitro* culture in a basal culture medium devoid of exogenous cytokines and growth factors and having a reactivity with specific antibodies selected from the group of SSEA-1 (TEC01) and EMA-1.

2. Avian stem cell line according to Claim 1, **characterized in that** said line has at least one of the following characteristics:
- a clearly visible nucleolus and a very weak nucleoplasm;
- endogenous telomerase activity;
- and endogenous alkaline phosphatase activity.

3. Cell line according to one of Claims 1 and 2, **characterized in that** step b) also comprises a gradual withdrawal of the foetal calf serum, said cells of said lines being able to proliferate in the absence of foetal calf serum and of exogenous cytokines and growth factors.

4. Cell line according to one of Claims 1 to 3, **characterized in that** step b) also comprises a gradual withdrawal of the feeder layer, said cells of said lines being able to proliferate in the absence of feeder layer and of exogenous cytokines and growth factors.

5. Avian cell line according to one of Claims 1 to 4, **characterized in that** the cells of said line are able to proliferate for at least 100 days.

6. Avian cell line according to one of Claims 1 to 4, **characterized in that** the cells of said line are able to proliferate for at least 150 days.

7. Avian cell line according to one of Claims 1 to 4, **characterized in that** the cells of said line are able to proliferate for at least 300 days.

8. Avian cell line according to one of Claims 1 to 4, **characterized in that** the cells of said line are able to proliferate for at least 600 days.

9. Avian cell line according to one of Claims 1 to 8, **characterized in that** said basal culture medium is selected from the group consisting of DMEM, GMEM, Ham's F12 and McCoy's, supplemented with non-essential amino acids, vitamins and sodium pyruvate.

10. Avian cell line according to one of Claims 1 to 9, **characterized in that** said cell line is a line of adherent cells.

11. Avian cell line according to one of Claims 1 to 9, **characterized in that** said cell line is a line of nonadherent cells which proliferate in suspension.

12. Avian cell line according to one of Claims 1 to 11, **characterized in that** said cells replicate a living or attenuated virus, said virus being selected from the group of adenoviruses, hepadnaviruses, herpesviruses, orthomyxoviruses, papovaviruses, paramyxoviruses, picomaviruses, poxviruses, reoviruses and retroviruses.

13. Avian cell line according to Claim 12, **characterized in that** said virus is an orthomyxovirus and is the influenza virus.

14. Avian cell line according to Claim 12, **characterized in that** said virus is a paramyxovirus selected from the group consisting of the measles, mumps and rubella viruses.

15. Avian cell line according to Claim 12, **characterized in that** said virus is a poxvirus selected from the group consisting of the canarypox and fowlpox viruses.
